# DEMANDE DE BREVET EUROPEEN

(11) **EP 1 082 972 A2**
(43) Date de publication de la demande: **14.03.2001**
(21) Numéro de dépôt: 00402128.3
(22) Date de dépôt: 25.07.2000
(51) Int. Cl.: A61M 16/06, A62B 18/00

(54) **Equipement d'oxygenothérapie avec dispositif d'assistance respiratoire sans tube nasal.**

(30) Priorité: 30.08.1999 FR 9910905
(71) Demandeur: L'AIR LIQUIDE, SOCIETE ANONYME POUR L'ETUDE ET L'EXPLOITATION DES PROCEDES GEORGES CLAUDE, 75321 Paris Cédex 07 (FR); Air Liquide Sante (International), 75341 Paris Cedex 07 (FR)
(72) Inventeur: Larquet, Christian, 78280 Guyancourt (FR); Marié, Bruno, 78180 Montigny le Bretonneux (FR); Villermet, Alain, 78220 Viroflay (FR)
(74) Mandataire: Le Moenner, Gabriel

(57) **Abrégé**

L'invention concerne un dispositif portable d'administration de gaz permettant d'alimenter au moins une narine (3) d'un utilisateur (2) avec au moins une partie d'un gaz, comprenant au moins une buse (1) de distribution de gaz et des moyens de maintien (5) permettant de supporter et/ou de maintenir en position au moins ladite buse (1) de distribution de gaz sur le visage de l'utilisateur et le long d'au moins une partie de la paroi ou surface (4) externe du nez ou des joues dudit utilisateur (2), lorsque le dispositif est positionné sur la tête et/ou le visage dudit utilisateur (2).

L'invention concerne, en outre, un procédé d'injection de gaz à un utilisateur, ainsi qu'un équipement d'oxygénothérapie.

## Description

L'invention concerne un dispositif d'administration de gaz ou de mélange gazeux, en particulier d'oxygène, par voie nasale à un utilisateur, tel un patient, un sportif ou un pilote d'avion par exemples.

De façon connue, dans le domaine médical, les appareils d'aide respiratoire comprennent généralement un générateur d'oxygène très concentré et des tubes nasaux.

Le générateur peut être soit une bouteille d'oxygène de qualité cryogénique et/ou pharmaceutique, soit un concentrateur d'oxygène, tel un dispositif à cycles PSA (Pressure Swing Adsorption) permettant de produire de l'oxygène à une pureté supérieure à 90% à partir d'air.

En sortie de générateur, l'oxygène circule dans des tubes d'une longueur, en général, d'environ un à trois mètres et, est injecté à travers une buse constituée de deux petits tubes de 10 à 12 mm de longueur, enfoncés dans les narines de manière à ce qu'il soit respiré par le patient.

Les patients déficients respiratoires doivent avoir ces petits tubes enfoncés dans leurs narines pendant des durées journalières variant, selon la gravité de leur maladie, entre 12 et 24 heures.

Or, des blessures ou irritations sont parfois engendrées par les petits tubes sur les parois nasales internes qui, sous l'effet additionné de l'injection d'oxygène très concentré (≥ 90%) et d'une vitesse élevée d'éjection du gaz, peuvent devenir très douloureuses.

Le but de la présente invention vise donc à résoudre le problème d'une administration efficace d'oxygène à un utilisateur, tel un patient, en particulier à un patient présentant un déficit respiratoire important, ne conduisant à aucune lésion des parois internes ou des cavités nasales de cet utilisateur ou patient, même lorsque celui-ci doit être alimenté en oxygène pendant des durées journalières longues pouvant atteindre 24 heures.

La solution apportée par la présente invention est alors un dispositif portable d'administration de gaz permettant d'alimenter au moins une narine d'un utilisateur avec au moins une partie d'un gaz, comprenant au moins une buse de distribution de gaz et des moyens de maintien permettant de supporter et/ou de maintenir en position au moins ladite buse de distribution de gaz à proximité ou au contact du visage de l'utilisateur et le long d'au moins une partie de la paroi ou surface externe du visage située à proximité de la région nasale dudit utilisateur, lorsque le dispositif est positionné sur la tête et/ou le visage dudit utilisateur.

L'invention concerne aussi un dispositif portable d'administration de gaz permettant d'alimenter au moins une narine d'un utilisateur avec au moins une partie d'un gaz, comprenant au moins une buse de distribution de gaz et des moyens de maintien permettant de supporter et/ou de maintenir en position au moins ladite buse de distribution de gaz à proximité ou au contact du visage de l'utilisateur et le long d'au moins une partie de la paroi ou surface nasale externe dudit utilisateur, lorsque le dispositif est positionné sur la tête et/ou le visage dudit utilisateur.

De plus, l'invention concerne également un dispositif portable d'administration de gaz permettant d'alimenter au moins une narine d'un utilisateur avec au moins une partie d'un gaz, comprenant au moins une buse de distribution de gaz et des moyens de maintien permettant de supporter et/ou de maintenir en position au moins ladite buse de distribution de gaz à proximité ou au contact du visage de l'utilisateur et le long d'au moins une partie de la paroi ou surface d'au moins une des joues de l'utilisateur et à proximité de la région nasale dudit utilisateur, lorsque le dispositif est positionné sur la tête et/ou le visage dudit utilisateur.

Selon le cas, le dispositif de l'invention peut comprendre l'une ou plusieurs des caractéristiques suivantes :
- au moins une buse de distribution de gaz est dirigée vers au moins une narine de l'utilisateur et est positionnée à l'extérieur de ladite au moins une narine, lorsque le dispositif est positionné sur la tête et/ou le visage de l'utilisateur. En d'autres termes, selon l'invention, la ou les buses de distribution de gaz ne pénètrent pas, même partiellement, à l'intérieur des narines de l'utilisateur. Dit autrement, celles-ci sont positionnées à proximité immédiate des narines, c'est-à-dire de la région nasale, soit au niveau des joues, soit au niveau des côtés longitudinaux du nez, et le flux gaz délivré par les buses chemine donc, au moins temporairement et/ou localement, à l'extérieur desdites narines avant d'y pénétrer lors d'une inhalation du gaz par l'utilisateur.
- il comporte, en outre, des moyens d'acheminement de gaz permettant d'acheminer le gaz jusqu'au moins ladite buse de distribution, de préférence lesdits moyens d'acheminement de gaz comprennent au moins une canalisation de gaz.
- les moyens d'acheminement de gaz comportent au moins une canalisation souple ou flexible, de préférence au moins une canalisation en polymère.
- il comporte au moins deux buses de distribution de gaz, de préférence lesdites buses de distribution sont agencées de manière à être positionnées de part et d'autre du nez de l'utilisateur, le long de la paroi nasale externe.
- les moyens d'acheminement de gaz comportent au moins une canalisation de gaz multiple formée d'une conduite externe et d'au moins une conduite interne,
- les moyens d'acheminement de gaz comportent au moins une canalisation de gaz multiple formée d'une canalisation externe et d'au moins une canalisation interne concentriques.
- les moyens de maintien sont choisis dans le groupe formé par les supports de types lunettes ou demi-lunettes, les dispositifs ayant une forme de nez artificiel, les bandeaux serre-tête et les dispositifs pince-nez.
- la ou les buses ont un diamètre ou une largeur compris entre 0.2 mm et 25 mm, de préférence entre 0.4 mm et 13 mm.
- la ou les buses ont une extrémité de sortie de gaz de forme cylindrique, ovale ou aplatie, de préférence une extrémité de sortie de forme aplatie.

Par ailleurs, l'invention concerne aussi un procédé pour administrer par voie nasale un gaz ou un mélange gazeux à un utilisateur, dans lequel ;
(a) on achemine ledit gaz ou mélange gazeux jusqu'à au moins une buse de distribution de gaz située à proximité ou au contact du visage de l'utilisateur et agencée le long d'au moins une partie de la paroi ou surface externe du visage située à proximité de la région nasale dudit utilisateur, notamment le long d'au moins une partie de la paroi nasale externe ou le long d'au moins une partie de la paroi ou surface d'au moins une des joues de l'utilisateur et à proximité de la région nasale dudit utilisateur ; et
(b) on délivre au moins un flux dudit gaz ou mélange gazeux au moyen de ladite au moins une buse de distribution en direction d'au moins une des narines de l'utilisateur, ledit flux gazeux venant balayer au moins une partie de la surface ou paroi externe du visage de l'utilisateur, notamment au moins une partie de la surface ou paroi externe du nez ou d'au moins une des joues de l'utilisateur.

De préférence, le gaz ou mélange gazeux est de l'oxygène ou un gaz contenant de l'oxygène.

Avantageusement, la vitesse du gaz ou mélange gazeux délivré par la buse de distribution est comprise entre 0,1 m/s et 10 m/s, de préférence inférieure à environ 5 m/s.

Préférentiellement, le flux gazeux est délivré par au moins deux buses de distribution.

En outre, l'invention concerne aussi un équipement d'administration de gaz par voie inhalatoire, en particulier utilisable dans le domaine médical, comprenant au moins une source de gaz reliée à au moins un dispositif selon l'invention, de préférence des moyens de régulation de débit de gaz sont agencés entre ladite source de gaz et ledit dispositif.

En particulier, il comprend, en outre des moyens d'humidification de gaz agencés entre les moyens de régulation de débit de gaz et la ou les buses.

Avantageusement, la source de gaz est un récipient de gaz de préférence une bouteille de gaz, ou un appareil concentrateur d'oxygène permettant de produire de l'oxygène ou un gaz riche en oxygène à partir d'air.

En effet, tout en respectant les caractéristiques en débit et concentration d'oxygène respiré par un patient, le système d'injection selon l'invention évite toute blessure au patient et apporte assurément une amélioration considérable des dispositifs et systèmes connus.

En effet, les dispositifs d'administration connus comportent classiquement un système de buses d'injection permettant de diriger le jet d'oxygène dans la ou les narines et, dès lors, de contrôler le flux d'oxygène entrant dans le système respiratoire du patient.

Habituellement, les buses sont des tubes disposés à l'intérieur des narines.

Afin d'éviter les blessures citées ci-dessus à l'intérieur de la cavité nasale, il peut être envisagé d'injecter l'oxygène au travers de buses placées à l'extérieur des narines.

Or, il est connu qu'un jet de gaz a tendance à s'évaser à l'air libre lors de sa progression, ce qui entraîne une diminution de sa vitesse moyenne et l'empêche d'atteindre partiellement ou complètement sa cible.

En effet, un jet d'oxygène injecté à l'air libre à l'aide d'une buse de forme quelconque ne peut atteindre entièrement sa "cible", c'est-à-dire une narine, que s'il répond aux conditions d'être très fin, de toujours posséder une vitesse élevée et d'être injecté très proche de la narine.

Ceci ne peut dès lors, être obtenu qu'avec une buse très fine placée à l'entrée de la narine, conformément à l'art antérieur connu.

Le jet d'oxygène, dont la vitesse est nécessairement élevée pour bien atteindre la cible, peut alors rebondir sur les parois nasales si la buse est légèrement mal orientée, entraînant une perte d'oxygène et surtout un mauvais contrôle de la quantité respirée.

En tout état de cause, la sensation de respirer un jet de forte vitesse injecté directement dans les narines est très désagréable.

Or, ceci ne se produit pas avec le dispositif de l'invention.

En effet, les études réalisées par les inventeurs montrent que, grâce au dispositif selon l'invention, il est possible de faire plaquer un jet de gaz sur une paroi convexe que constitue, par exemple, la paroi externe de la ou des narines de telle manière qu'il suive cette paroi jusqu'à l'intérieur de la narine et qu'il puisse être respiré par l'utilisateur ou, selon un autre exemple, par la paroi ou la surface externe de la joue située à proximité de la ou des narines.

Les caractéristiques du jet doivent pour cela se situer dans un domaine très précis, en termes notamment de vitesse et dimensions.

Dès lors, les inventeurs de la présente invention ont montré qu'un jet pariétal d'oxygène injecté, par exemple, le long du nez (et non directement à l'intérieur des narines) peut, s'il est bien orienté et si ses caractéristiques sont en conformité avec les courbures du nez, atteindre complètement les narines pour être respiré par le patient ou l'utilisateur.

Une telle solution permet à la fois d'éviter l'introduction de tubes dans les narines et de contrôler le débit d'oxygène respiré par le patient ou l'utilisateur, donc de répondre efficacement au problème ci-dessus.

La présente invention va maintenant être mieux comprise grâce à un exemple de réalisation et aux figures annexées, donnés à titre illustratif mais non limitatif.

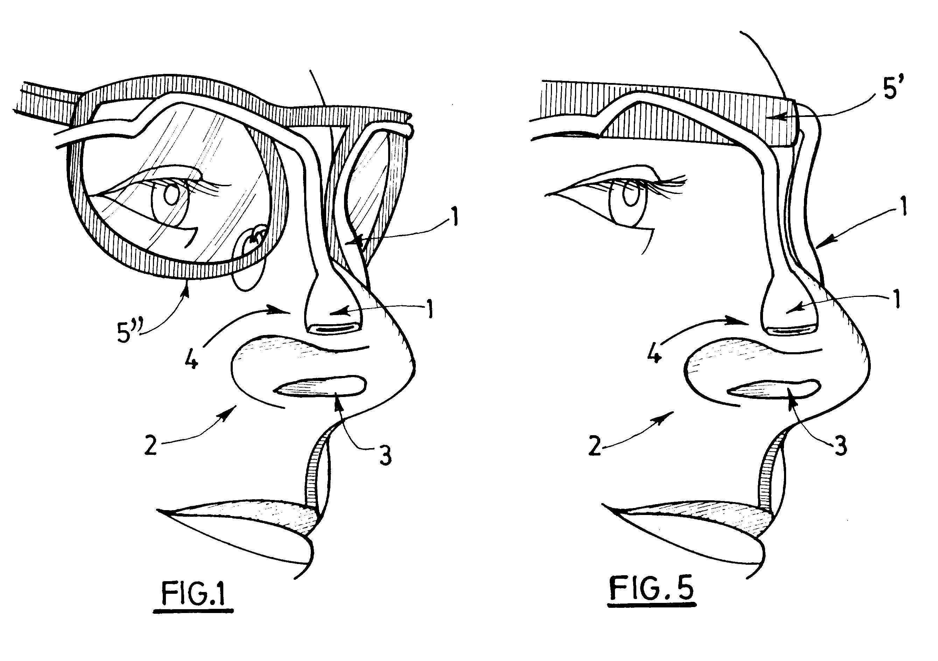

Les figures 1 et 3 à 5 représentent des schémas d'un dispositif d'administration de gaz conforme à l'invention, comprenant deux buses d'injection 1 d'oxygène à un utilisateur 2.

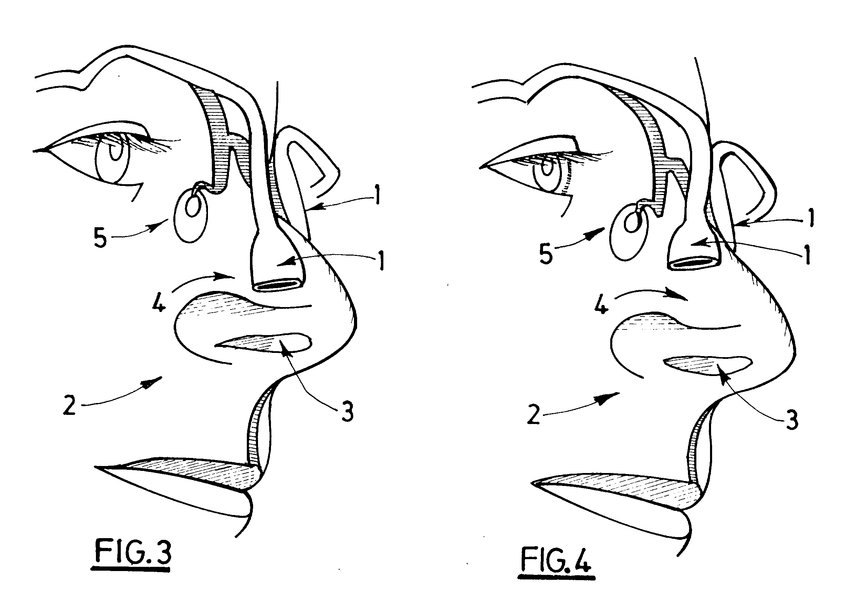

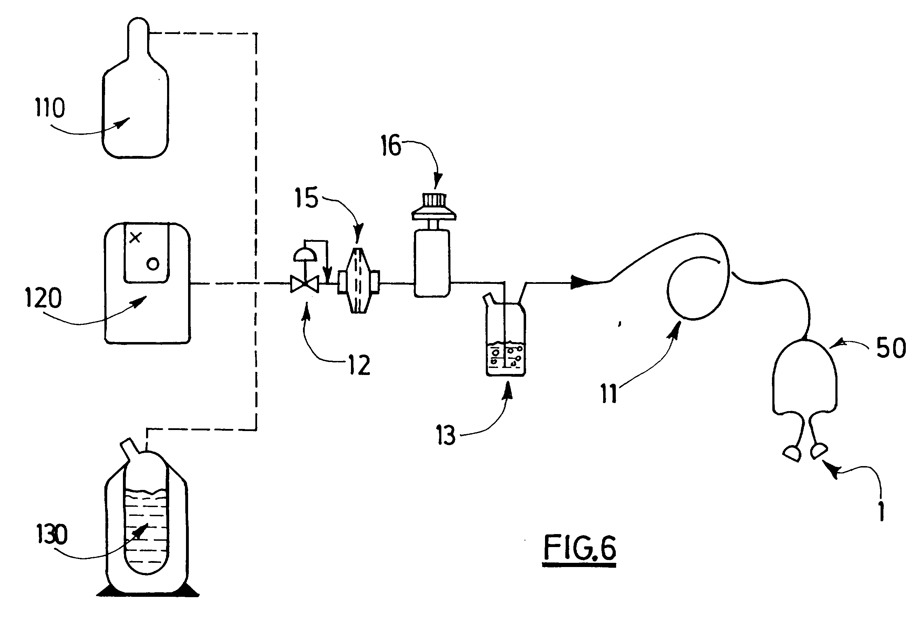

Comme on peut le voir, les buses 1 sont agencées le long de la paroi externe 4 du nez de l'utilisateur 2, et de part et d'autre de celui-ci, c'est-à-dire de chaque côté de l'arête nasale, en y étant maintenue en position par des moyens de maintien adaptés, tels qu'une ou plusieurs sangles ou bandeau 5' serre-tête (Figure 5), une structure ou une armature pouvant venir se positionner sur la tête de l'utilisateur, un dispositif du type lunettes 5" (Figure 1) ou demi-lunettes (Figures 3 et 4) venant prendre appui sur les oreilles et/ou le nez de l'utilisateur 2 ou analogue.

Selon un autre mode de réalisation (non représenté), la buse peut également être solidaire d'un dispositif de type "faux nez", c'est-à-dire un nez artificiel venant se substituer soit au moins à la partie inférieure de la paroi ou surface externe du nez, c'est-à-dire la région située à l'extrémité du nez proche des narines, soit au moins à la partie supérieure de la paroi ou surface externe du nez, c'est-à-dire la région située à la base du nez et entre les yeux, de manière à permettre, dans un cas comme dans l'autre, d'uniformiser les caractéristiques et l'orientation de la ou des buses quelle que soit la forme du nez du patient.

Le faux-nez peut intégrer les buses, c'est-à-dire que celles-ci peuvent être fixées à ce faux-nez ou intégré dans la structure même de celui-ci, directement par moulage par exemple.

L'oxygène injecté, sensiblement de haut en bas, chemine le long de la paroi extérieure 4 du nez avant de pénétrer à l'intérieur du nez par les narines 3.

Afin de vérifier l'efficacité du dispositif selon l'invention, une étude a été menée pour mettre au point les conditions d'obtention du contrôle complet de la quantité d'oxygène respirée.

Pour ce faire :
. le visage de l'utilisateur a été simulé par un masque reproduisant un visage humain ;
. les narines du masque ont été équipées de tubes reliés à une pompe et un analyseur d'oxygène permettant à la fois de simuler la respiration de l'utilisateur et de connaître la quantité d'oxygène effectivement respirée ;
. une buse d'injection a été placée en différents endroits du nez afin de connaître la précision nécessaire de l'emplacement et son influence sur la quantité d'oxygène respirée ;
. en maintenant un débit de respiration constant, à savoir un débit simulant le débit de respiration humaine pendant la phase inspiratoire c'est-à-dire environ 24 l/min. Le débit d'injection a été modifié au cours du temps et différentes dimensions de buse ont été testées afin de connaître le domaine de fonctionnement (dimensions, vitesse) dans lequel l'ensemble de l'oxygène injecté est respiré ;
. enfin, un ventilateur placé à des distances variées du masque ont permis d'étudier l'attachement et le contrôle du jet d'oxygène dans des conditions atmosphériques extérieures (présence de vent).

Les résultats obtenus montrent qu'il est possible de bien contrôler le débit d'oxygène respiré lorsque la vitesse du gaz est maintenue inférieure à 10 m/s, de préférence inférieure à environ 5 m/s.

Au-delà, une partie de l'oxygène injecté n'est pas respiré par le patient.

Toutefois, dans tous les cas, la majeure partie de l'oxygène injecté est respiré par le patient.

Une remarque importante concerne la présence ou non de vent.

En effet, en absence de ventilateur simulant le vent, la position de la buse d'injection sur le nez n'influence que très peu les performances du système.

En revanche, en présence de vent (de l'ordre de quelques m/s), c'est-à-dire un ventilateur placé à 1.50 mètre par exemple, le contrôle est mal assuré si la buse est placée en haut du nez (au niveau des supports de type lunettes). Toutefois, si la buse est placée dans la partie basse du nez, le contrôle est parfaitement assuré jusqu'à environ 5 m/s.

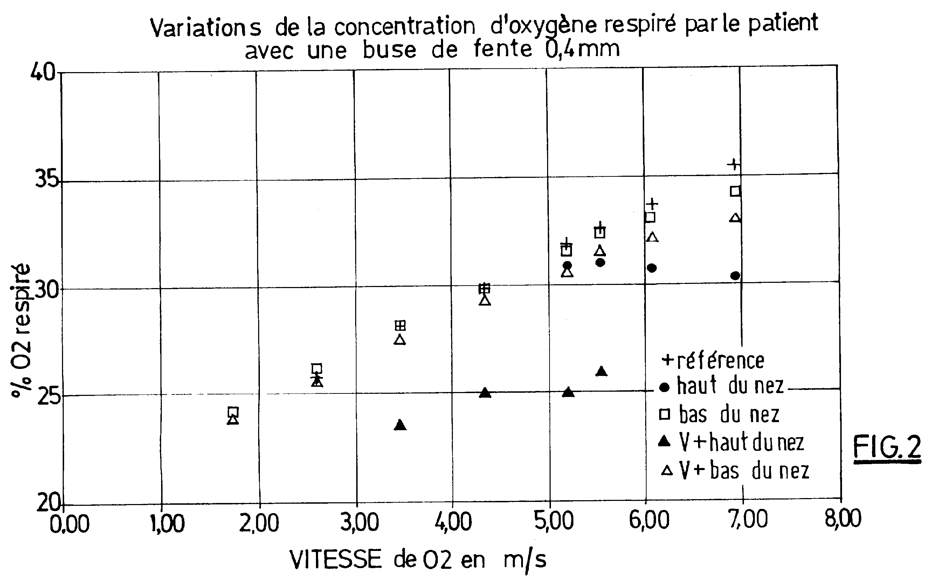

La figure 2, ci-jointe, montre clairement le pourcentage d'oxygène respiré par l'utilisateur, simulé par le système pompe-analyseur d'oxygène, en fonction de la vitesse d'injection de l'oxygène pour différentes configurations représentées sur les figures 3 et 4 :
. référence : correspond au pourcentage d'oxygène obtenu lorsque l'injection d'oxygène est effectuée à l'intérieur même du tube de respiration. Cette référence correspond à une respiration de 100% de l'oxygène injecté ;
. haut du nez : la buse d'injection est placée sur la partie haute du nez, à l'endroit où se trouvent habituellement des supports de type lunettes (fig. 4) ;
. bas du nez : la buse d'injection est placée sur la partie basse du nez, à l'endroit de la courbure de la paroi verticale nasale (fig. 3) ;
. V + Haut du nez : la buse d'injection est placée sur la partie haute du nez en présence d'un ventilateur placé à environ 1.50 mètre.
. V + Bas du nez : la buse d'injection est placée sur la partie basse du nez en présence d'un ventilateur placé à environ 1.50 mètre.

La figure 2 confirme qu'il est possible de contrôler efficacement le débit d'oxygène respiré par le patient lorsque la buse est placée dans la partie basse du nez, et ceci même en présence d'un fort vent.

Pour toutes ces configurations, la buse est la même, c'est-à-dire une buse de forme aplatie, la hauteur de sa fente de sortie étant de 0.4 mm et sa largeur d'environ 1.25 cm. Le débit correspondant à environ 5 m/s (la vitesse limite du contrôle total) est de 1.5 l/min pour chaque buse. Les essais sur d'autres hauteurs de fente sont similaires et la vitesse limite semble se situer toujours vers environ 5 m/s.

Cette dernière remarque est importante car elle implique que les résultats présentés peuvent être obtenus avec des débits très différents, la vitesse de l'oxygène injecté étant le paramètre important de ce système, à condition que le jet injecté reste suffisamment fin pour se comporter comme un jet pariétal.

Ainsi, avec une buse de fente 0.8 mm de hauteur et d'environ 1.25 cm de largeur, le contrôle total d'oxygène respiré par l'utilisateur a été obtenu jusqu'à un débit d'environ 3 l/min pour chaque buse pour une vitesse limite d'environ 5 m/s..

La figure 6 représente, quant à elle, un équipement d'oxygénothérapie selon l'invention comportant une source de gaz pouvant être : soit une bouteille d'oxygène gazeux 110, soit un générateur d'air enrichi en oxygène, c'est-à-dire un concentrateur d'oxygène 120, soit un récipient contenant de l'oxygène liquide 130.

Cette source de gaz est reliée, par l'intermédiaire d'une canalisation 11 de gaz, à un dispositif selon l'invention, tel que montré sur les figures 1 et 3 à 5.

On voit sur cette figure 6 que, d'une part, des moyens de régulation de pression, tel un détendeur 12 de gaz, et des moyens de régulation de débit de gaz, tel un sélecteur de débit 16, sont agencés entre la source d'oxygène et le dispositif selon l'invention.

En outre, on constate aussi la présence possible, sur la ligne 11, d'un filtre bactériologique 15 et d'un humidificateur 13 de gaz est situé, entre le détendeur 12 et le dispositif 50 à buses 1 selon l'invention.

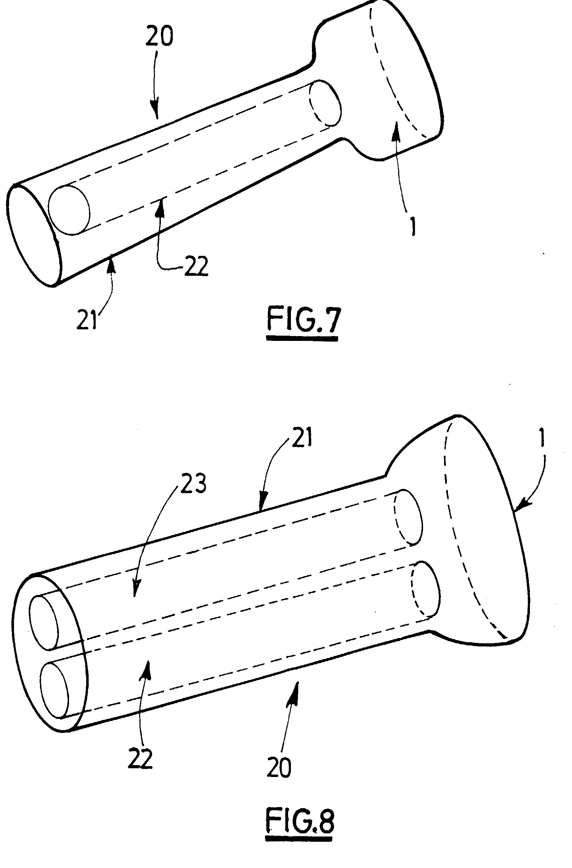

En outre, la figure 7 schématise un mode de réalisation particulier de l'invention selon lequel la buse 1 de distribution de gaz est alimentée en deux gaz de nature et/ou de composition différentes acheminées par une canalisation multiple 20 formée, par exemple, d'une conduite externe 21 et d'une conduite interne 22 ; de manière à permettre d'administrer des gaz différents à l'utilisateur.

La figure 8 est semblable à la figure 7 à l'exception du fait que, dans ce cas, la buse 1 est alimentée en gaz par une canalisation multiple 20 formée d'une conduite externe 21 et de plusieurs conduites internes 22 et 23, juxtaposées et/ou concentriques.

L'invention est particulièrement adaptée au domaine médical mais peut également être appliquée à d'autres fins, par exemple dans le domaine du sport ou celui de l'aéronautique, lorsqu'un sportif ou un pilote d'avion peut avoir besoin temporairement d'une assistance en oxygène.

## Revendications

1. Dispositif portable d'administration de gaz permettant d'alimenter au moins une narine d'un utilisateur (2) avec au moins une partie d'un gaz, comprenant au moins une buse (1) de distribution de gaz et des moyens de maintien (5) permettant de supporter et/ou de maintenir en position au moins ladite buse (1) de distribution de gaz à proximité ou au contact du visage de l'utilisateur (2) et le long d'au moins une partie de la paroi ou surface externe du visage située à proximité de la région nasale dudit utilisateur (2), lorsque le dispositif est positionné sur la tête et/ou le visage dudit utilisateur.

2. Dispositif portable d'administration de gaz permettant d'alimenter au moins une narine d'un utilisateur (2) avec au moins une partie d'un gaz, comprenant au moins une buse (1) de distribution de gaz et des moyens de maintien (5) permettant de supporter et/ou de maintenir en position au moins ladite buse (1) de distribution de gaz à proximité ou au contact du visage de l'utilisateur (2) et le long d'au moins une partie de la paroi ou surface nasale (4) externe dudit utilisateur (2), lorsque le dispositif est positionné sur la tête et/ou le visage dudit utilisateur.

3. Dispositif portable d'administration de gaz permettant d'alimenter au moins une narine d'un utilisateur (2) avec au moins une partie d'un gaz, comprenant au moins une buse (1) de distribution de gaz et des moyens de maintien (5) permettant de supporter et/ou de maintenir en position au moins ladite buse (1) de distribution de gaz à proximité ou au contact du visage de l'utilisateur (2) et le long d'au moins une partie de la paroi ou surface d'au moins une des joues de l'utilisateur et à proximité de la région nasale dudit utilisateur (2), lorsque le dispositif est positionné sur la tête et/ou le visage dudit utilisateur.

4. Dispositif selon l'une des revendications 1 à 3, caractérisé en ce qu'au moins une buse (1) de distribution de gaz est dirigée vers au moins une narine de l'utilisateur et est positionnée à l'extérieur de ladite au moins une narine, lorsque le dispositif est positionné sur la tête et/ou le visage de l'utilisateur.

5. Dispositif selon l'une des revendications 1 à 4, caractérisé en ce qu'il comporte, en outre, des moyens d'acheminement de gaz permettant d'acheminer le gaz jusqu'au moins ladite buse (1) de distribution, de préférence lesdits moyens d'acheminement de gaz comprennent au moins une canalisation de gaz.

6. Dispositif selon l'une des revendications 1 à 5, caractérisé en ce que les moyens d'acheminement de gaz comportent au moins une canalisation souple ou flexible, de préférence au moins une canalisation en polymère.

7. Dispositif selon l'une des revendications 1 à 6, caractérisé en ce qu'il comporte au moins deux buses (1) de distribution de gaz, de préférence lesdites buses (1) de distribution sont agencées de manière à être positionnées de part et d'autre du nez de l'utilisateur, le long de la paroi (4) nasale externe.

8. Dispositif selon l'une des revendications 1 à 7, caractérisé en ce que les moyens d'acheminement de gaz comportent au moins une canalisation de gaz multiple formée d'une conduite externe et d'au moins une conduite interne.

9. Dispositif selon l'une des revendications 1 à 8, caractérisé en ce que les moyens de maintien sont choisis dans le groupe formé par les supports de types lunettes (5) ou demi-lunettes, les dispositifs ayant une forme de nez artificiel, les bandeaux serre-tête et les dispositifs pince-nez.

10. Dispositif selon l'une des revendications 1 à 9, caractérisé en ce que la ou les buses (1) ont un diamètre ou une largeur compris entre 0.2 mm et 25 mm, de préférence entre 0.4 mm et 13 mm.

11. Dispositif selon l'une des revendications 1 à 10, caractérisé en ce que la ou les buses ont une extrémité de sortie de gaz de forme cylindrique, ovale ou aplatie, de préférence une extrémité de sortie de forme aplatie.

12. Procédé pour administrer par voie nasale un gaz ou un mélange gazeux à un utilisateur, en dehors du domaine thérapeutique, dans lequel :
(a) on achemine ledit gaz ou mélange gazeux jusqu'à au moins une buse de distribution de gaz située à proximité ou au contact du visage de l'utilisateur et agencée le long d'au moins une partie de la paroi ou surface externe du visage située à proximité de la région nasale dudit utilisateur (2), notamment le long d'au moins une partie de la paroi nasale externe ou le long d'au moins une partie de la paroi ou surface d'au moins une des joues de l'utilisateur et à proximité de la région nasale dudit utilisateur (2) ; et
(b) on délivre au moins un flux dudit gaz ou mélange gazeux au moyen de ladite au moins une buse de distribution en direction d'au moins une des narines de l'utilisateur, ledit flux gazeux venant balayer au moins une partie de la surface ou paroi externe du visage de l'utilisateur, notamment au moins une partie de la surface ou paroi externe du nez ou d'au moins une des joues de l'utilisateur.

13. Procédé selon la revendication 12, caractérisé en ce que le gaz ou mélange gazeux est de l'oxygène ou un gaz contenant de l'oxygène.

14. Procédé selon l'une des revendications 12 ou 13, caractérisé en ce que la vitesse du gaz ou mélange gazeux délivré par la buse de distribution est comprise entre 0,1 m/s et 10 m/s, de préférence inférieure à environ 5 m/s.

15. Procédé selon l'une des revendications 12 à 14, caractérisé en ce que le flux gazeux est délivré par au moins deux buses de distribution.

16. Equipement d'administration de gaz par voie inhalatoire, en particulier utilisable dans le domaine médical, comprenant au moins une source de gaz reliée à au moins un dispositif selon l'une des revendications 1 à 11, de préférence des moyens de régulation de débit de gaz sont agencés entre ladite source de gaz et ledit dispositif.

17. Equipement selon la revendication 16, caractérisé en ce qu'il comprend, en outre, des moyens d'humidification de gaz agencés entre les moyens de régulation de débit de gaz et la ou les buses.

18. Equipement selon l'une des revendication 16 ou 17, caractérisé en ce que la source de gaz est un récipient de gaz, de préférence une bouteille de gaz, ou un appareil concentrateur d'oxygène permettant de produire de l'oxygène ou un gaz riche en oxygène à partir d'air.
